(19) Europäisches Patentamt
European Patent Office
Office européen des brevets

(11) EP 4 166 600 A1

(12) EUROPEAN PATENT APPLICATION
published in accordance with Art. 153(4) EPC

(43) Date of publication:
**19.04.2023 Bulletin 2023/16**

(21) Application number: **22739823.7**

(22) Date of filing: **18.01.2022**

(51) International Patent Classification (IPC):
***C08J 3/24*** (2006.01) ***C08J 3/12*** (2006.01)
***C08J 3/075*** (2006.01) ***C08J 9/14*** (2006.01)
***C08F 265/06*** (2006.01) ***C08F 220/06*** (2006.01)
***C08F 2/44*** (2006.01) ***C08K 9/10*** (2006.01)

(52) Cooperative Patent Classification (CPC):
**C08F 2/44; C08F 220/06; C08F 265/06; C08J 3/075; C08J 3/12; C08J 3/24; C08J 9/14; C08K 9/10**

(86) International application number:
**PCT/KR2022/000861**

(87) International publication number:
**WO 2022/154631 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2021 KR 20210006720**
**17.01.2022 KR 20220006530**

(71) Applicant: **LG CHEM, LTD.**
**Yeongdeungpo-gu,**
**Seoul 07336 (KR)**

(72) Inventors:
- **KIM, Ju Eun**
  **Daejeon 34122 (KR)**
- **YANG, Seung Hun**
  **Daejeon 34122 (KR)**
- **CHO, Joonil**
  **Daejeon 34122 (KR)**
- **LEE, Serin**
  **Daejeon 34122 (KR)**
- **LEE, Hoyong**
  **Daejeon 34122 (KR)**
- **LEE, Jungmin**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) METHOD FOR PREPARING SUPERABSORBENT POLYMER

(57) Provided are a superabsorbent polymer composition, and a preparation method thereof. More particularly, provided are a superabsorbent polymer, of which a high absorption rate and excellent gel strength are improved at the same time by using specific additives in combination during polymerization, and a preparation method thereof.

EP 4 166 600 A1

## Description

[Technical Field]

Cross-reference to Related Application

The present application is based on, and claims priority from, Korean Patent Application Nos. 10-2021-0006720 and 10-2022-0006530, filed on January 18, 2021 and January 17, 2022, respectively, the disclosures of which are hereby incorporated by reference herein in their entirety.

The present invention relates to a superabsorbent polymer, of which a high absorption rate and excellent gel strength are improved at the same time by using specific additives in combination during polymerization, and a preparation method thereof.

[Background Art]

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in sanitary products, now they have been widely used not only for hygiene products such as disposable diapers for children, etc., but also for water retaining soil products for gardening, water stop materials for the civil engineering and construction, sheets for raising seedling, fresh-keeping agents for food distribution fields, materials for poultice or the like.

In most cases, these superabsorbent polymers have been widely used in the field of hygienic materials such as diapers, sanitary pads, etc. For these applications, superabsorbent polymers are required to exhibit high absorbency with respect to water, etc., and to exhibit excellent absorption performances under pressure not to release the absorbed water even under an external pressure.

In addition, when the superabsorbent polymer is included in hygienic materials such as diapers, etc., it is necessary to spread urine as wide as possible even in an environment pressurized by a user's weight. Through this, it is possible to further improve the absorption performances and absorption rate of hygienic materials by wholly utilizing superabsorbent polymer particles contained in the entire area of the hygienic material absorption layer. Further, due to such spreading characteristics under pressure, it is possible to further improve the rewetting property of diapers, which prevents urine once absorbed by the superabsorbent polymer from being re-exuded. In addition, it is possible to improve the leakage suppression characteristic of diapers. Previously, attempts have been made to improve the characteristics of widely spreading urine by changing the design of hygienic materials such as diapers, etc. Recently, as hygienic materials become thinner and the content of superabsorbent polymer in hygienic materials is relatively increased, the improvement of the spreading characteristic by design changes of the hygienic materials themselves is limited.

When the superabsorbent polymer is included in hygienic materials such as diapers, etc., it comes into direct contact with a user's skin. Thus, when urine absorbed by the superabsorbent polymer is not absorbed quickly, the residue will come into contact with the skin for a long time, causing a problem such as reduction in the comfort of wearing, skin irritation, etc. Accordingly, it is necessary to exhibit a higher absorption rate. In addition, when practically used in products such as hygienic materials, etc., polymer chains in the superabsorbent polymer are broken by components such as ascorbic acid present in urine. Thus, as the wearing time increases, mechanical properties of the polymer, i.e., gel strength, are greatly deteriorated, causing a problem that overall physical properties are also deteriorated.

Accordingly, in consideration of conditions when products are practically used, it is necessary to study a superabsorbent polymer capable of realizing an appropriate gel strength while maintaining excellent absorption properties.

[Disclosure]

[Technical Problem]

Provided are a superabsorbent polymer, of which a high absorption rate and excellent gel strength are improved at the same time, while maintaining excellent absorption properties by using three kinds of additives in combination during polymerization, and a preparation method thereof.

[Technical Solution]

To achieve the above object, provided is a method of preparing a superabsorbent polymer, the method including the steps of:

forming a water-containing gel polymer including a crosslinked polymer obtained by crosslinking polymerization of a water-soluble ethylenically unsaturated monomer having an acidic group of which at least a part is neutralized, in the presence of an epoxy-based internal crosslinking agent, a chelating agent, and a capsule-type foaming agent;
drying the water-containing gel polymer to form a base polymer powder; and
surface-crosslinking the base polymer powder by heat treatment in the presence of a surface crosslinking agent,
wherein the epoxy-based internal crosslinking agent has a weight average molecular weight of 200 kDa to 1,000 kDa, and
the capsule-type foaming agent has a structure including a core including hydrocarbon and a shell surrounding the core, the shell formed by a thermoplastic resin.

[Effect of the Invention]

According to a method of preparing a superabsorbent polymer of the present invention, it is possible to provide a superabsorbent polymer, of which a high absorption rate and excellent gel strength are improved at the same time, while exhibiting excellent absorption performances.

[Best Mode for Carrying Out the Invention]

The terms used in this description are just for explaining exemplary embodiments and it is not intended to restrict the present invention.

The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components or combinations thereof beforehand.

In the present invention, the terms "the first", "the second", "the third" and the like are used to describe a variety of components, and these terms are merely employed to differentiate one component from other components.

The present invention may be variously modified and have various forms, and specific embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

As used herein, the term "polymer" means a polymerized state of water-soluble ethylenically unsaturated monomers, and may encompass those of all water content ranges or particle size ranges. Among the polymers, those having a water content (a moisture content) of about 40% by weight or more after being polymerized and before being dried may be called a water-containing gel polymer. Particles obtained by pulverizing and drying the water-containing gel polymer may be called a crosslinked polymer.

Further, the term "superabsorbent polymer powder" refers to a particle phase material including a crosslinked polymer which is obtained by polymerizing and crosslinking a water-soluble ethylenically unsaturated monomer having an acidic group of which at least a part is neutralized, in the presence of an internal crosslinking agent.

Further, the "superabsorbent polymer" refers to, depending on the context, a crosslinked polymer obtained by polymerizing a water-soluble ethylenically unsaturated monomer having an acidic group of which at least a part is neutralized, or a base polymer in the form of powder, consisting of superabsorbent polymer particles obtained by pulverizing the crosslinked polymer, or is used to encompass those made suitable for commercialization by an additional process of the crosslinked polymer or the base polymer, for example, surface crosslinking, reassembling of fine particles, drying, pulverizing, size-sorting, etc.

As used herein, the term "internal crosslinking agent" is used to distinguish it from a "surface crosslinking agent" which crosslinks the surface of the base polymer, and the internal crosslinking agent functions to polymerize the water-soluble ethylenically unsaturated monomers by crosslinking the unsaturated bonds thereof. The crosslinking in the above step occurs regardless of the surface or inside of the polymer. However, through the surface crosslinking process of the base polymer described below, the particle surface of the finally prepared superabsorbent polymer has a structure crosslinked by the surface crosslinking agent, and the inside thereof has a structure crosslinked by the internal crosslinking agent.

Hereinafter, a method of preparing a superabsorbent polymer and the superabsorbent polymer according to specific embodiments of the present invention will be described in more detail.

The method of preparing a superabsorbent polymer according to one embodiment of the present invention includes the steps of forming a water-containing gel polymer including a crosslinked polymer obtained by crosslinking polymerization of a water-soluble ethylenically unsaturated monomer having an acidic group of which at least a part is neutralized, in the presence of an epoxy-based internal crosslinking agent, a chelating agent, and a capsule-type foaming

agent; drying the water-containing gel polymer to form a base polymer powder; and surface-crosslinking the base polymer powder by heat treatment in the presence of a surface crosslinking agent.

The superabsorbent polymer is a hygienic material and may exist in a swollen state by the urine, body fluid, etc. for a long time when actually used. In this case, there has been a problem in that the polymer chains are destroyed by accelerating the action of residual radicals inside the polymer by the ascorbic acid component in the urine and body fluid, and accordingly, the gel strength of the polymer significantly decreases as the wearing time increases in the swollen state. As a result, overall absorption properties are also deteriorated due to changes of the internal structure.

Accordingly, the present inventors found that, when three kinds of additives are used in combination during polymerization of superabsorbent polymers, their synergistic effect may provide a superabsorbent polymer having excellent overall absorption performances, such as water retention capacity, absorbency under pressure, etc., a very high initial absorption rate, and at the same time, further improved surface feeling due to excellent gel strength, leading to the present invention.

Specifically, it was found that when the specific capsule-type foaming agent of the present invention is used, the superabsorbent polymer has a sufficiently large surface area, thereby exhibiting excellent initial absorbency. As a result, the superabsorbent polymer may provide hygienic materials, such as diapers, sanitary pads, etc., capable of quickly absorbing body fluids and giving a dry feel. In addition, when a specific epoxy-based crosslinking agent and a chelating agent are used in combination, a new crosslinking structure is formed by chemically binding to the main chain of the polymer and the structure formed by the capsule-type foaming agent and the chelating agent during polymerization, and it is possible to realize excellent absorbency under pressure and gel strength by the above structure.

Accordingly, the prepared superabsorbent polymer exhibits changes in the degree of gel contraction and water flow characteristics in response to an external pressure in the state of absorbing the body fluid. Due to this structure, the superabsorbent polymer may exhibit improved absorption rate and gel strength.

Hereinafter, each step of the method of preparing a superabsorbent polymer according to specific embodiments of the present invention will be described in more detail.

The method of preparing a superabsorbent polymer according to specific embodiments of the present invention includes the step of forming a water-containing gel polymer including a crosslinked polymer obtained by crosslinking polymerization of a water-soluble ethylenically unsaturated monomer having an acidic group of which at least a part is neutralized, in the presence of an epoxy-based internal crosslinking agent, a chelating agent, and a capsule-type foaming agent.

In the above crosslinking polymerization, crosslinking polymerization of a monomer composition including components generally used in the preparation of superabsorbent polymers in addition to the above ingredients may be performed.

First, the water-soluble ethylenically unsaturated monomer may be any monomer commonly used in the preparation of superabsorbent polymers. Non-limiting examples of the water-soluble ethylenically unsaturated monomer may be a compound represented by the following Chemical Formula 1:

[Chemical Formula 1] $R_1\text{-COOM}^1$

in Chemical Formula 1,

$R_1$ is an alkyl group containing an unsaturated bond and having 2 to 5 carbon atoms, and
$M^1$ is a hydrogen atom, a monovalent or divalent metal, an ammonium group, or an organic amine salt.

Preferably, the monomer may include one or more selected from the group consisting of acrylic acid, methacrylic acid, and a monovalent metal salt thereof, a divalent metal salt thereof, an ammonium salt thereof, and an organic amine salt thereof. As described, when acrylic acid or a salt thereof is used as the water-soluble ethylenically unsaturated monomer, it is advantageous in terms of obtaining a superabsorbent polymer having improved absorbency. In addition, the monomer may include one or more selected from the group consisting of an anionic monomer such as maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, and a salt thereof; a nonionic hydrophilic monomer such as (meth)acrylamide, N-substituted (meth)acrylate, 2-hydroxyethyl(meth)acrylate, 2-hydroxypropyl(meth)acrylate, methoxypolyethyleneglycol(meth)acrylate, or polyethyleneglycol(meth)acrylate; and an unsaturated monomer containing an amino group such as (N,N)-dimethylaminoethyl(meth)acrylate or (N,N)-dimethylaminopropyl(meth)acrylamide, and a quaternary compound thereof.

Here, the water-soluble ethylenically unsaturated monomer may have an acidic group, in which at least a part of the acidic group may be neutralized. Preferably, those partially neutralized with an alkaline substance such as sodium hydroxide, potassium hydroxide, ammonium hydroxide or the like may be used as the monomer.

In this regard, the degree of neutralization of the monomer may be 40 mol% to 95 mol%, or 40 mol% to 80

mol%, or 45 mol% to 75 mol%. The range of the degree of neutralization may vary depending on the final physical properties. An excessively high degree of neutralization causes the neutralized monomers to be precipitated, and thus polymerization may not readily occur. On the contrary, an excessively low degree of neutralization not only deteriorates the absorbency of the polymer but also endows the polymer with hard-to-handle properties, like elastic rubber.

In the superabsorbent polymer of one embodiment, the water-containing gel polymer may include a crosslinked polymer obtained by polymerization of the above-described water-soluble ethylenically unsaturated monomer in the presence of an epoxy-based internal crosslinking agent, a chelating agent, and a capsule-type foaming agent.

The epoxy-based internal crosslinking agent is a component that introduces a new crosslinking structure to the polymer, and in particular, it contributes to the improvement of gel strength by forming a uniform network. As the epoxy-based internal crosslinking agent, an epoxy-based compound having a weight average molecular weight of 200 kDa to 1,000 kDa is used. Preferably, an epoxy-based compound of 300 kDa to 800 kDa, or 500 kDa to 700 kDa is used.

When the weight average molecular weight of the epoxy-based internal crosslinking agent is less than 200 kDa, it is difficult to achieve a high water retention capacity, and when it exceeds 1,000 kDa, there is a problem in that the water retention capacity is excessively increased.

Meanwhile, the weight average molecular weight is measured by gel permeation chromatography (GPC, PL GPC220, Agilent Technologies) under the following conditions:

- Column: PL Olexis(Polymer Laboratories)
- Solvent: Trichlorobenzene (TCB)
- Flow rate: 1.0 ml/min
- Sample concentration: 1.0 mg/ml
- Input amount: 200 μl
- Column temperature: 160°C
- Detector: Agilent High Temperature RI detector
- Standard: Polystyrene (calibrated by a cubic function)

The epoxy-based internal crosslinking agent may be a compound having an epoxy equivalent weight of 100 g/eq to 400 g/eq, preferably, an epoxy equivalent weight of 110 g/eq to 380 g/eq. When the epoxy equivalent weight of the epoxy-based crosslinking agent is less than 100 g/eq, there may be a problem in that flexibility of the crosslinked polymer network is lowered and the absorbency of the superabsorbent polymer is lowered. On the contrary, when the epoxy equivalent weight is as high as 400 g/eq or more, it may be difficult to form a uniform crosslinked structure.

Specific examples of the epoxy-based internal crosslinking agent include ethylene glycol diglycidyl ether, diethylene glycol diglycidyl ether, poly(ethylene glycol)n diglycidyl ether (n=4 to 13) or a combination thereof. In addition, the type of commercially available epoxy-based internal crosslinking agents may include JSI's EJ-1030, EJ-831, EJ-841, etc., but is not limited thereto.

The epoxy-based internal crosslinking agent may be used in an amount of 0.01 part by weight to 10 parts by weight, 0.03 parts by weight to 5 parts by weight, or 0.05 parts by weight to 3 parts, based on 100 parts by weight of the water-soluble ethylenically unsaturated monomer. In the above content range, the epoxy-based internal crosslinking agent may be used in combination with the chelating agent and the capsule-type foaming agent to be described later to maximize the synergistic effect.

The chelating agent is a component contributing to the effect of improving gel strength by binding to metal ions during the crosslinking polymerization of the polymer. In particular, the interaction with $Fe^{2+}$ prevents oxidation of the polymer by metal ions in the novel crosslinked structure.

The chelating agent is one or more selected from the group consisting of ethylenediamine tetraacetic acid (EDTA), diethylenetriamine pentaacetic acid (DTPA), phenolic acid, citric acid, amino acids and salts thereof. Preferably, ethylenediamine tetraacetic acid (EDTA), diethylenetriamine pentaacetic acid (DTPA), or a sodium salt thereof may be used, and more preferably, an ethylenediamine tetraacetic acid (EDTA) tetrasodium salt or diethylenetriamine pentaacetic acid (DTPA) may be used.

The chelating agent may be used in an amount of 0.01 part by weight to 10 parts by weight, 0.03 parts by weight to 5 parts by weight, 0.1 part by weight to 3 parts by weight, based on 100 parts by weight of the water-soluble ethylenically unsaturated monomer. In the above content range, the chelating agent may be used in combination with the epoxy-based internal crosslinking agent and the capsule-type foaming agent to maximize the synergistic effect.

The capsule-type foaming agent exists in an encapsulated state in the polymer during polymerization, and is foamed by heat applied during a drying process to be described later. As a result, pores with an appropriate size are generated between the polymer structures of the superabsorbent polymer and allow the superabsorbent polymer sheet to exhibit an open pore channel structure. In particular, it is possible to significantly improve the gel strength of the polymer by interaction with the epoxy-based internal crosslinking agent.

The encapsulated foaming agent of the monomer composition may have a structure including a core including

hydrocarbon and a shell surrounding the core, the shell formed by a thermoplastic resin. Such an encapsulated foaming agent may have expansion properties which may vary depending on components constituting the core and the shell, weights of the respective components, diameters thereof. By adjusting these factors, it is possible to expand pores to a desired size and to control porosity of the superabsorbent polymer sheet.

When pores with an appropriate size are generated on the surface of the polymer particles, it is possible to greatly improve the initial absorbency of the superabsorbent polymer. Specifically, it is preferable that the particle size of the polymer particles is 300 μm to 425 μm, and the size of the pores is about 20 μm to about 200 μm. When the above-described encapsulated foaming agent is used, it is possible to prepare polymer particles having pores with such a size on the surface thereof. To prepare a large amount of the polymer particles having pores with the appropriate size, it is necessary to examine expansion properties of the encapsulated foaming agent. However, the foamed shape of the encapsulated foaming agent inside the superabsorbent polymer is difficult to define as one shape, because it may vary depending on the preparation conditions of the superabsorbent polymer. Therefore, the encapsulated foaming agent is first foamed in air, and then its expansion ratio and size are examined, thereby determining whether it is suitable for the preparation of polymer particles having pores with the appropriate size.

In detail, the encapsulated foaming agent is applied on a glass petri dish, which is then heated in air at 150 °C for 10 minutes to expand the encapsulated foaming agent. In this regard, when the encapsulated foaming agent exhibits a maximum expansion ratio of 3 times to 15 times, 5 times to 15 times, or 8.5 times to 10 times in air, it may be determined as being suitable for forming an appropriate open pore structure in the method of preparing the superabsorbent polymer sheet of the present invention.

The encapsulated foaming agent may have a mean diameter of 5 μm to 50 μm, or 5 μm to 30 μm, or 5 μm to 20 μm, or 7 μm to 17 μm. When the encapsulated foaming agent exhibits the above mean diameter, it may be determined as being suitable for achieving an appropriate porosity.

Further, when the encapsulated foaming agent exhibits a maximum expansion diameter of 20 μm to 190 μm, or 50 μm to 190 μm, or 70 μm to 190 μm, or 75 μm to 190 μm in air, it may be determined as being suitable for forming an appropriate open pore structure in the method of preparing the superabsorbent polymer sheet of the present invention.

The hydrocarbon constituting the core of the encapsulated foaming agent may be one or more selected from the group consisting of n-propane, n-butane, iso-butane, cyclobutane, n-pentane, iso-pentane, cyclopentane, n-hexane, iso-hexane, cyclohexane, n-heptane, iso-heptane, cycloheptane, n-octane, iso-octane, and cyclooctane. Among them, hydrocarbons having 3 to 5 carbon atoms (n-propane, n-butane, iso-butane, cyclobutane, n-pentane, iso-pentane, cyclopentane) may be suitable for forming the pores with the above-described size, and iso-butane may be the most suitable.

The thermoplastic resin constituting the shell of the encapsulated foaming agent may be a polymer formed from one or more monomers selected from the group consisting of (meth)acrylate, (meth)acrylonitrile, aromatic vinyl, vinyl acetate, vinyl halide, and vinylidene halide. Among them, a copolymer of (meth)acrylate and (meth)acrylonitrile may be the most suitable for forming the pores with the above-described size.

The encapsulated foaming agent may include the hydrocarbon in an amount of 10% by weight to 30% by weight, based on the total weight of the encapsulated foaming agent. This range may be the most suitable for forming the open pore structure.

As the encapsulated foaming agent, a directly prepared encapsulated foaming agent may be used, or a commercially available foaming agent satisfying the above-described conditions may be used. The commercially available encapsulated foaming agent may include Matsumoto's F-36D, Dongjin Semichem's MS-140DS, but is not limited thereto.

Further, the encapsulated foaming agent may be used in an amount of 0.01 part by weight to 10 parts by weight, 0.03 parts by weight to 5 parts by weight, 0.1 part by weight to 3 parts by weight, based on 100 parts by weight of the water-soluble ethylenically unsaturated monomer. When the content of the encapsulated foaming agent is too low, there is a problem in that the open pore structure may not be properly formed. When the content of the encapsulated foaming agent is too high, porosity may be too high, and as a result, strength of the superabsorbent polymer may be weakened. In this point of view, the encapsulated foaming agent may be preferably used in the above range of content.

Meanwhile, the crosslinked polymer may be subjected to crosslinking polymerization by further including a branched polymer of poly(meth)acrylic acid including polyalkylene glycol in side chains. In other words, the branched polymer may be further included in the monomer composition. The branched polymer is poly(meth)acrylic acid including polyalkylene glycol in side chains, and may contribute to improving gel strength by physically crosslinking the inside of the water-containing gel polymer during the preparation of the superabsorbent polymer.

Preferably, the branched polymer may be a copolymer in which polyethylene glycol and/or methoxypolyethylene glycol branches are bound to a poly(meth)acrylic acid linear polymer.

More preferably, the branched polymer may be a copolymer including a repeating unit represented by the following Chemical Formula 1-1 and a repeating unit represented by the following Chemical Formula 1-2:

[Chemical Formula 1-1]

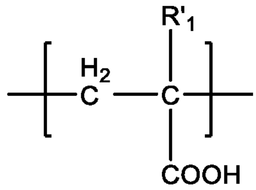

in Chemical Formula 1-1,
R'$_1$ may be each independently hydrogen or methyl,

[Chemical Formula 1-2]

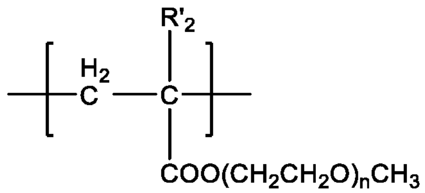

in Chemical Formula 1-2,

R'$_2$ may be each independently hydrogen or methyl, and
n may be an integer of 1 to 100.

More preferably, the branched polymer may be a copolymer including a repeating unit represented by the following Chemical Formula 1-3:

[Chemical Formula 1-3]

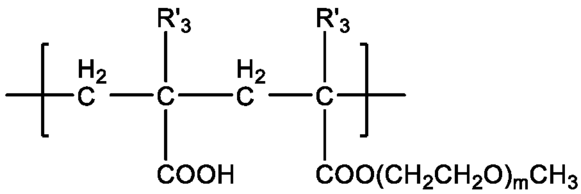

in Chemical Formula 1,

R'$_3$ may be each independently hydrogen or methyl, and
m may be an integer of 1 to 100.

Preferably, n and m may be each independently 1 to 50, more preferably 5 to 30, and most preferably 5 to 15.
On the other hand, the branched polymer has a weight average molecular weight of 10,000 g/mol to 100,000 g/mol, 20,000 g/mol to 50,000 g/mol, or 20,000 g/mol to 30,000 g/mol, as measured by gel permeation chromatography (GPC: PL GPC220, Agilent Technologies) using PS standards.
Preferably, the branched polymer may be used in an amount of 0.01 part by weight to 10 parts by weight, based on 100 parts by weight of the water-soluble ethylenically unsaturated monomer. When the branched polymer is used in an amount of less than 0.01 part by weight, based on 100 parts by weight of the water-soluble ethylenically unsaturated monomer, the branched polymer is difficult to perform the role of improving gel strength, and it is used in an amount of more than 10 parts, the physical properties of the prepared superabsorbent polymer may deteriorate.
In the crosslinking polymerization, a polymerization initiator generally used in the preparation of superabsorbent polymers may be included. Non-limiting examples of the polymerization initiator may include a thermal polymerization initiator or a photopolymerization initiator, depending on a polymerization method. Particularly, a thermal polymerization

initiator may be used. However, even though the photopolymerization is performed, a certain amount of heat may be generated by UV irradiation or the like, and also generated with exothermic polymerization reaction. Therefore, the thermal polymerization initiator may be further included.

As the thermal polymerization initiator, one or more compounds selected from the group consisting of persulfate-based initiators, azo-based initiators, hydrogen peroxide, and ascorbic acid may be used. Specifically, the persulfate-based initiators may include sodium persulfate ($Na_2S_2O_8$), potassium persulfate ($K_2S_2O_8$), ammonium persulfate ($(NH_4)_2S_2O_8$) or the like. The azo-based initiators may include 2,2-azobis(2-amidinopropane) dihydrochloride, 2,2-azo-bis-(N,N-dimethylene)isobutyramidine dihydrochloride, 2-(carbamoylazo)isobutylonitrile, 2,2-azobis[2-(2-imidazolin-2-yl)propane] dihydrochloride, 4,4-azobis-(4-cyanovaleric acid) or the like. More various thermal polymerization initiators are disclosed in "Principle of Polymerization (Wiley, 1981)" written by Odian, p203, which may be served as a reference.

The photopolymerization initiator may include, for example, one or more compounds selected from the group consisting of benzoin ether, dialkyl acetophenone, hydroxyl alkyl ketone, phenyl glyoxylate, benzyl dimethyl ketal, acyl phosphine, and $\alpha$-aminoketone. Among them, specific examples of the acylphosphine may include diphenyl(2,4,6-trimethylbenzoyl)phosphine oxide, phenylbis(2,4,6-trimethylbenzoyl)phosphine oxide, ethyl (2,4,6-trimethylbenzoyl)phenylphosphinate or the like. More various photopolymerization initiators are well disclosed in "UV Coatings: Basics, Recent Developments and New Application(Elsevier 2007)" written by Reinhold Schwalm, p 115, but are not limited thereto. The polymerization initiator may be added at a concentration of about 0.0001% by weight to 1% by weight, or 0.001% by weight to 1% by weight, based on the monomer composition including the water-soluble ethylenically unsaturated monomer. When the thermal polymerization initiator and the photopolymerization initiator are used at the same time, the above content means the total content of the mixture thereof.

In other words, when the concentration of the polymerization initiator is too low, the polymerization rate may become slow and a large amount of residual monomer may be extracted in the final product, which is not preferred. On the contrary, when the concentration of the polymerization initiator is too high, polymer chains constituting the network become short, and thus the content of water-soluble components is increased and physical properties of the polymer may deteriorate, such as a reduction in absorbency under pressure, which is not preferred.

In addition, the monomer composition may further include additives such as a surfactant, a thickener, a plasticizer, a preservation stabilizer, an antioxidant, as needed.

The surfactant induces uniform dispersion of the foaming agent and prevents the gel strength from being lowered or the density from being lowered due to uniform foaming during foaming. An anionic surfactant is preferably used as the surfactant. Specifically, the surfactant includes $SO_3^-$ anion, and a compound represented by the following Chemical Formula 2 may be used.

[Chemical Formula 2] $R\text{-}SO_3Na$

in Chemical Formula 2,

R is an alkyl having 8 to 16 carbon atoms.

In addition, the surfactant is preferably used in an amount of 300 ppmw or less, based on the weight of the water-soluble ethylenically unsaturated monomer. When the amount of the surfactant exceeds 300 ppmw, the content of the surfactant in the superabsorbent polymer increases, which is not preferred. Further, the surfactant is preferably used in an amount of 100 ppmw or more, or 150 ppmw or more, based on the weight of the water-soluble ethylenically unsaturated monomer.

The monomer composition may be prepared in the form of a solution, in which the raw materials, such as the above-described monomer, polymerization initiator, internal crosslinking agent, etc., are dissolved in a solvent.

In this regard, as the solvent to be applicable, any solvent may be used without limitations in the constitution as long as it is able to dissolve the above components. For example, as the solvent, water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, N,N-dimethylacetamide, or a mixture thereof may be used.

Further, formation of the water-containing gel polymer through polymerization of the monomer composition may be carried out by a common polymerization method, and a process thereof is not particularly limited. For non-limiting example, the polymerization method is largely classified into thermal polymerization and photopolymerization according to a polymerization energy source. The thermal polymerization may be carried out in a reactor like a kneader equipped with agitating spindles. The photopolymerization may be carried out in a reactor equipped with a movable conveyor belt.

For example, the monomer composition is injected to the reactor like the kneader equipped with the agitating spindles, and thermal polymerization is carried out by providing hot air thereto or by heating the reactor, thereby obtaining the water-containing gel polymer. In this regard, the water-containing gel polymer may be obtained as particles having a size of centimeters or millimeters when it is discharged from an outlet of the reactor, according to the type of agitating

spindles equipped in the reactor. Specifically, the water-containing gel polymer may be obtained in various forms according to a concentration of the monomer composition fed thereto, a feeding speed or the like, and the water-containing gel polymer having a weight average particle size of 2 mm to 50 mm may be generally obtained.

For another example, when the monomer composition is subjected to photopolymerization in the reactor equipped with the movable conveyor belt, the water-containing gel polymer may be obtained in a sheet-type. In this regard, the thickness of the sheet may vary according to the concentration of the monomer composition fed thereto and the feeding speed. The sheet is preferably controlled to have a thickness of 0.5 cm to 10 cm in order to assure the production speed while allowing the entire sheet to be uniformly polymerized.

The water-containing gel polymer formed by the method may exhibit a water content of 40% by weight to 80% by weight. Meanwhile, the "water content" means a weight occupied by water with respect to the total weight of the water-containing gel polymer, which may be a value obtained by subtracting the weight of the dried polymer from the weight of the water-containing gel polymer. Specifically, the water content may be defined as a value calculated by measuring the weight loss due to evaporation of moisture in the polymer during the process of drying by raising the temperature of the polymer through infrared heating. At this time, the water content may be measured under the drying conditions of maintaining from room temperature to about 180°C for 40 minutes.

Next, the method of preparing a superabsorbent polymer according to one embodiment of the present invention includes the step of drying the prepared water-containing gel polymer to form a base polymer powder. Through the drying step, the capsule-type foaming agent is foamed, and pores with the appropriate size are generated between the polymer structures of the superabsorbent polymer, and thus the superabsorbent polymer sheet gives the open pore channel structure, thereby improving the absorption rate of the superabsorbent polymer.

Meanwhile, before drying, the step of coarsely pulverizing may be performed, as needed, in order to increase efficiency of the drying process.

In this regard, a pulverizer to be applicable may include, but the constitution is not limited, specifically, any one selected from the group consisting of a vertical pulverizer, a turbo cutter, a turbo grinder, a rotary cutter mill, a cutter mill, a disc mill, a shred crusher, a crusher, a chopper, and a disc cutter, but is not limited thereto.

In this regard, the pulverization may be performed so that the polymer has a particle size of about 2 mm to about 10 mm.

To pulverize the polymer at a particle size of less than 2 mm is technically not easy due to the high water content of the water-containing gel polymer, and agglomeration may occur between the pulverized particles. On the contrary, when the polymer is pulverized at a particle size of more than 10 mm, the effect of increasing the efficiency in the subsequent drying step may become insignificant.

The polymer pulverized as above or immediately after polymerization without the pulverization is subjected to a drying process.

The drying temperature of the drying step may be about 150°C to about 250°C. When the drying temperature is lower than 150°C, the foaming agent is not sufficiently foamed, the drying time becomes excessively long, and the physical properties of the superabsorbent polymer finally formed may be deteriorated. When the drying temperature is higher than 250°C, only the surface of the polymer is excessively dried, and thus fine powder may be generated during the subsequent pulverization process, and the physical properties of the superabsorbent polymer finally formed may be deteriorated. Preferably, the drying may be carried at a temperature of about 150°C to about 230°C, and more preferably at a temperature of about 180°C to about 200°C. Meanwhile, the drying time is about 10 minutes to about 120 minutes, preferably, about 20 minutes to 90 minutes, in view of process efficiency, but is not limited thereto.

With regard to the drying method of the drying step, any method may be selected and used without limitation in the constitution as long as it may be commonly used for drying the water-containing gel polymer. Specifically, the drying step may be carried out by a method of supplying hot air, irradiating infrared rays, irradiating microwaves, irradiating ultraviolet rays or the like. The water content of the polymer after carrying out the drying step may be about 5% by weight to about 10% by weight.

According to one embodiment of the present invention, the step of pulverizing the dried polymer obtained through the drying step may be carried out.

The base polymer which is the polymer powder obtained after the pulverization may have a particle size of 150 μm to 850 μm. A pulverizer used to pulverize into such a particle size may specifically include a pin mill, a hammer mill, a screw mill, a roll mill, a disc mill, a jog mill, or the like, but the present invention is not limited thereto.

In order to manage physical properties of the superabsorbent polymer powder finally produced after the pulverization, the base polymer obtained after the pulverization is size-sorted. Preferably, polymers having a particle size of about 150 μm to about 850 μm are size-sorted, and only the base polymer having such a particle size may be subjected to a surface crosslinking reaction.

Meanwhile, according to one embodiment of the present invention, after forming the base polymer powder, the step of surface-crosslinking the base polymer powder by heat treatment in the presence of a surface crosslinking agent is carried out.

The surface crosslinking step is to form the surface-crosslinked layer using the surface crosslinking agent in order to increase the surface crosslinking density of the base polymer, and unsaturated bonds of the water-soluble ethylenically unsaturated monomers, which remain uncrosslinked on the surface, may be crosslinked by the surface crosslinking agent, and as a result, superabsorbent polymer particles having an increased surface crosslinking density may be formed. Through this heat treatment process, the surface crosslinking density, i.e., the external crosslinking density increases, whereas the internal crosslinking density does not change. Therefore, the superabsorbent polymer, in which the surface-crosslinked layer is formed, may have a structure in which the external crosslinking density is higher than the internal crosslinking density.

In the method of one embodiment, a surface crosslinking agent composition including a surface crosslinking agent, an alcohol-based solvent, and water may be used in the step of forming the surface-crosslinked layer.

Meanwhile, as the surface crosslinking agent included in the surface crosslinking agent composition, surface crosslinking agents which have been used in the preparation of the superabsorbent polymers may be used without particular limitation. For example, the surface crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol, and glycerol; one or more carbonate-based compounds selected from the group consisting of ethylene carbonate and propylene carbonate; epoxy compounds such as ethylene glycol diglycidyl ether, etc.; oxazoline compounds such as oxazolidinone, etc.; polyamine compounds; oxazoline compounds; mono-, di-, or polyoxazolidinone compounds; or cyclic urea compounds, etc. Preferably, those the same as the above-described internal crosslinking agent may be used, and for example, a diglycidyl ether-based compound of alkylene glycol, such as ethyleneglycol diglycidyl ether, etc., may be used.

Such a surface crosslinking agent may be used in an amount of 0.001 part by weight to 2 parts by weight, based on 100 parts by weight of the base polymer. For example, the surface crosslinking agent may be used in an amount of 0.005 parts by weight or more, 0.01 part by weight or more, or 0.02 parts by weight or more, and 0.5 parts by weight or less, 0.3 parts by weight or less, based on 100 parts by weight of the base polymer. By controlling the content range of the surface crosslinking agent within the above-described range, it is possible to prepare a superabsorbent polymer exhibiting overall physical properties, such as excellent absorption performances, liquid permeability, etc.

Meanwhile, the surface crosslinked layer may further include an additive. For example, caking may be suppressed by further including aluminum sulfate and/or silica as the additive. Aluminum sulfate uniformly included in the surface crosslinked layer may further improve the overall strength of the superabsorbent polymer particles, and thus it is expected that liquid permeability may be improved because the shape of the particles may be maintained even after absorbing liquid.

Meanwhile, the superabsorbent polymer according to an embodiment of the present invention may have a particle size of 150 μm to 850 μm. More specifically, at least 95% by weight or more of the base polymer powder and the superabsorbent polymer including the same has a particle size of 150 μm to 850 μm. Particles having a particle size of 300 μm to 600 μm may be included in an amount of 50% by weight or more, and fine powder having a particle size of less than 150 μm may be included in an amount of less than 3% by weight.

Further, the superabsorbent polymer according to one embodiment of the present invention may provide a superabsorbent polymer having a higher absorption rate and excellent gel strength at the same time while maintaining excellent basic absorption performances such as water retention capacity, etc.

In detail, 2.5 g of the superabsorbent polymer is immersed in 50 g of ascorbic acid saline and allowed to swell in an oven at 40 °C for 24 hours. The swollen superabsorbent polymer has a gel strength of 0.50 N or more, as measured using a tensile and compression testing machine. Preferably, the gel strength is 0.50 N to 0.9 N, preferably, 0.6 N to 0.85 N or 0.65 N to 0.8 N. The ascorbic acid saline refers to an aqueous solution of 0.9 wt% sodium chloride (NaCl) and 0.005 wt% ascorbic acid.

The gel strength is an index indicating the decomposition resistance to L-ascorbic acid component contained in urine, body fluid, etc., and the measurement method thereof will be described in more detail in Experimental Examples to be described later.

The superabsorbent polymer has an absorption rate of 50 seconds or less according to the vortex method. Preferably, the absorption rate may be 48 seconds or less, or about 46 seconds or less, or 43 seconds or less. The absorption rate is excellent as the value is smaller, and therefore, the lower limit of the absorption rate is 0 second in theory. For example, the absorption rate may be about 10 seconds or more, or about 15 seconds or more, or about 20 seconds or more.

The absorption rate is measured in seconds according to a method described in International Patent Publication No. 1987-003208. In detail, the absorption rate (or vortex time) is measured as follows: 2 g of the superabsorbent polymer is added to 50 mL of physiological saline at 23°C to 24±1°C, followed by stirring using a magnetic bar (8 mm in diameter, 31.8 mm in length) at 600 rpm. The time until the vortex disappears is measured in seconds. As the time is shorter, the superabsorbent polymer may be determined to have a higher initial absorption rate.

In addition, the centrifuge retention capacity (CRC) is 36.5 g/g or more, preferably 36.5 g/g to 45.0 g/g, or 36.5 g/g to 40.0 g/g, as measured according to the EDENA method WSP 241.2. The method of measuring the centrifuge retention capacity will be described in more detail in Experimental Example to be described later.

The absorbency under pressure (AUP) of 0.7 psi is 17 g/g or more, as measured according to the EDANA method WSP 242.3-10. Preferably, the absorbency under pressure is 17.0 g/g to 21.0 g/g, 17.5 g/g or more, or 18.0 g/g or more. The method of measuring the absorbency under pressure (AUP) will be described in more detail in Experimental Example to be described later.

The superabsorbent polymer obtained according to the above-described preparation method may provide a superabsorbent polymer having a higher absorption rate and excellent gel strength at the same time while maintaining excellent basic absorption performances such as water retention capacity, etc.

Hereinafter, the actions and effects of the present invention will be described in more detail with reference to specific exemplary embodiments of the present invention. However, these exemplary embodiments are provided only for illustrating the present invention, and the scope of the present invention is not limited thereto.

**[Example]**

**<Preparation of Superabsorbent Polymer>**

**Example 1**

100 g of acrylic acid, 123.5 g of 32% caustic soda (NaOH), 0.2 g of sodium persulfate as a thermal polymerization initiator, 0.008 g of diphenyl(2,4,6-trimethylbenzoyl)-phosphine oxide as a photopolymerization initiator, 0.06 g of ethylene glycol diglycidyl ether, 0.1 g of an encapsulated foaming agent (F-36D, SDI Korea), 0.09 g of a 28% aqueous sodium dodecyl sulfate solution, 59 g of water, 0.3 g of a branched polymer containing a repeating unit of the following Chemical Formula 3'-1, and 0.1 g of EDTA-4Na were mixed in a 3-L glass container equipped with a stirrer, a nitrogen feeder, and a thermometer to prepare a monomer composition having a total solid content of 43% by weight. The monomer composition was fed at a feed rate of 500 mL/min to 2,000 mL/min onto a rotary belt having a width of 10 cm and a length of 2 m and rotating at a speed of 50 cm/min. Simultaneously with the feeding of the monomer composition, ultraviolet rays with an intensity of 10 mW/$cm^2$ were irradiated, and a polymerization reaction was allowed for 60 seconds.

[Chemical Formula 3'-1]

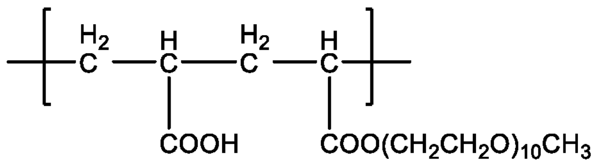

After completion of the polymerization reaction, the product was cut with a meat chopper and dried at 185 °C for 40 minutes using an air-flow oven.

To 100 g of the prepared base polymer powder, a mixed solution of 4 g of ultrapure water, 0.5 g of propylene glycol, 0.10 g of an epoxy-based surface crosslinking agent (EJ-1030, JSI Co.), 0.2 g of aluminum sulfate, and 0.06 g of Aerosil200 was added, followed by mixing for 2 minutes. The mixture was dried at 140 °C for 40 minutes, and size-sorted to obtain particles having a size of 150 μm ~ 850 μm. Thus, a superabsorbent polymer was prepared.

**Examples 2 to 9**

Each superabsorbent polymer composition was prepared in the same manner as in Example 1, except that components of the polymerization step were used as in the components and contents of Table 1 below.

**Comparative Example 1**

100 g of acrylic acid, 123.5 g of 32% caustic soda (NaOH), 0.2 g of sodium persulfate as a thermal polymerization initiator, 0.008 g of diphenyl(2,4,6-trimethylbenzoyl)-phosphine oxide as a photopolymerization initiator, 0.2 g of diacrylate, 0.1 g of an encapsulated foaming agent (F-36D, SDI Korea), 0.09 g of a 28% aqueous sodium dodecyl sulfate solution, and 59 g of water were mixed in a 3-L glass container equipped with a stirrer, a nitrogen feeder, and a thermometer to prepare a monomer composition having a total solid content of 43% by weight. The monomer composition was fed

at a feed rate of 500 mL/min to 2,000 mL/min onto a rotary belt having a width of 10 cm and a length of 2 m and rotating at a speed of 50 cm/min. Simultaneously with the feeding of the monomer composition, ultraviolet rays with an intensity of 10 $mW/cm^2$ were irradiated, and a polymerization reaction was allowed for 60 seconds.

After completion of the polymerization reaction, the product was cut with a meat chopper and dried at 185 °C for 40 minutes using an air-flow oven.

To 100 g of the prepared base polymer powder, a mixed solution of 7 g of ultrapure water, 0.5 g of propylene glycol, 0.10 g of an epoxy-based surface crosslinking agent (EJ-1030, JSI Co.), 0.8 g of aluminum sulfate, and 0.04 g of Aerosil200 was added, followed by mixing for 2 minutes. The mixture was dried at 140 °C for 40 minutes, and size-sorted to obtain particles having a size of 150 μm ~ 850 μm. Thus, a superabsorbent polymer was prepared.

**Comparative Examples 2 to 3**

Each superabsorbent polymer composition was prepared in the same manner as in Comparative Example 1, except that components of the polymerization step were used as in the components and contents of Table 1 below.

**Comparative Examples 4 and 5**

Each superabsorbent polymer composition was prepared in the same manner as in Example 1, except that components of the polymerization step were used as in the components and contents of Table 1 below.

[Table 1]

| Section | Polymerization crosslinking agent | | | |
|---|---|---|---|---|
| | Epoxy-based internal crosslinking agent (type/ content*) | Chelating agent (type/content*) | Capsule-type foaming agent (type/content*) | Other additives (type/content*) |
| Example 1 | A1/0.06 | B1/0.1 | C1/0.1 | - |
| Example 2 | A2/0.18 | B1/0.1 | C1/0.1 | - |
| Example 3 | A3/0.30 | B1/0.1 | C1/0.1 | - |
| Example 4 | A1/0.06 | B1/0.5 | C1/0.1 | - |
| Example 5 | A1/0.06 | B1/0.1 | C1/0.4 | - |
| Example 6 | A1/0.06 | B2/0.3 | C1/0.1 | - |
| Example 7 | A1/0.06 | B1/0.1 | C2/0.3 | - |
| Example 8 | A1/0.005 | B1/0.15 | C2/0.3 | |
| Example 9 | A1/0.40 | B1/15 | C1/20 | |
| Comparative Example 1 | - | - | C1/0.1 | D1/0.2 |
| Comparative Example 2 | - | - | C1/0.15 | D1/0.2 |
| Comparative Example 3 | - | - | C1/0.2 | D1/0.2 |
| Comparative Example 4 | A1/0.06 | B1/0.1 | - | - |
| Comparative Example 5 | A1/0.06 | - | C1/0.1 | - |
| * Based on 100 parts by weight of a water-soluble ethylenically unsaturated monomer<br>A1: Ethylene glycol diglycidyl ether (an epoxy equivalent weight: 113 g/eq, a weight average molecular weight: 200 kDa)<br>A2: Poly(ethylene glycol)$_5$ diglycidyl ether (an epoxy equivalent weight: 268 g/eq, a weight average molecular weight: 600 kDa) | | | | |

(continued)

| Section | Polymerization crosslinking agent | | | |
|---|---|---|---|---|
| | Epoxy-based internal crosslinking agent (type/ content*) | Chelating agent (type/content*) | Capsule-type foaming agent (type/content*) | Other additives (type/content*) |
| A3: Poly(ethylene glycol)$_{10}$ diglycidyl ether (an epoxy equivalent weight: 372 g/eq, a weight average molecular weight: 800 kDa)<br>B1: EDTA-4Na<br>B2: DTPA<br>C1: F-36D, Matsumoto<br>C2: MS-140DS, Dongjin Semichem<br>D1: Diacrylate | | | | |

**<Experimental Example>**

For the superabsorbent polymer compositions prepared in Examples and Comparative Examples, physical properties were evaluated by the following methods, and the results are shown in Table 2.

Unless otherwise indicated, all of the following physical properties were evaluated at room temperature (25 °C), and physiological saline or saline means a 0.9% wt aqueous sodium chloride (NaCl) solution. The ascorbic acid saline means an aqueous solution of 0.9 wt% sodium chloride (NaCl) and 0.005 wt% ascorbic acid.

(1) Centrifuge retention capacity (CRC)

Of the superabsorbent polymers, superabsorbent polymers having a particle size of 150 μm to 850 μm were taken, and the centrifuge retention capacity (CRC) by absorbency under no load was measured in accordance with European Disposables and Nonwovens Association (EDANA) standard EDANA WSP 241.2.

In detail, from the polymers obtained through Examples and Comparative Examples, a polymer size-sorted using a #30-50 sieve was obtained, respectively. The polymer $W_0$(g) (about 0.2 g) was evenly put in a nonwoven fabric-made bag, followed by sealing. Then, the bag was immersed in a physiological saline solution (0.9 wt %) at room temperature. After 30 minutes, water was removed from the bag using a centrifuge at 250 G for 3 minutes, and the weight $W_2$(g) of the bag was then measured. Further, the same procedure was carried out without using the polymer, and then the resultant weight $W_1$(g) was measured. By using the respective weights thus obtained, CRC (g/g) was calculated according to the following equation.

[Equation 1]

$$\text{CRC (g/g)} = \{[W_2(g) - W_1(g)]/W_0(g)\} - 1$$

(2) Absorbency under pressure (AUP)

The absorbency under pressure of 0.7 psi of each polymer was measured according to the EDANA method WSP 242.3-10. When the absorbency under pressure was measured, the size-sorted polymer used in the CRC measurement was used.

In detail, a 400 mesh stainless steel net was installed in the bottom of a plastic cylinder having an internal diameter of 25 mm. The superabsorbent polymer W0(g) (0.16 g) was evenly scattered on the steel net at room temperature and humidity of 50%, and a piston which may evenly provide a load of 0.7 psi was put thereon, in which an external diameter of the piston was slightly smaller than 25 mm, there was no gap between the internal wall of the cylinder and the piston, and the jig-jog of the cylinder was not interrupted. At this time, the weight W3(g) of the apparatus was measured.

After putting a glass filter having a diameter of 90 mm and a thickness of 5 mm in a Petri dish having a diameter of 150 mm, a physiological saline solution composed of 0.9 wt% sodium chloride was poured until the surface level of the physiological saline solution became equal to the upper surface of the glass filter. A sheet of filter paper having a diameter of 90 mm was put thereon. The measurement apparatus was mounted on the filter paper, thereby having the liquid absorbed under the load for 1 hour. 1 hour later, the weight $W_4$(g) was measured after lifting the measurement apparatus.

By using the respective weights thus obtained, absorbency under pressure(g/g) was calculated according to the following equation.

[Equation 2]

$$AUP(g/g) = [W_4(g) - W_3(g)]/W_0(g)$$

(3) Absorption rate (Vortex, sec)

Of the superabsorbent polymers, superabsorbent polymers having a particle size of 150 μm to 850 μm were taken, and 2 g of the superabsorbent polymer was added to 50 mL of physiological saline at 24±1°C, and stirred with a magnetic bar (8 mm in diameter and 31.8 mm in length) at 600 rpm, and a time taken for vortex to disappear was measured in seconds.

(4) Gel strength (N)

Of the superabsorbent polymers, superabsorbent polymers having a particle size of 150 μm to 850 μm were taken, and 2.5 g of the superabsorbent polymer was immersed in 50 g of ascorbic acid saline, and allowed to swell in an oven at 40 °C for 24 hours. The gel strength of the swollen superabsorbent polymer was measured using a tensile and compression testing machine.

In detail, the gel strength of the swollen superabsorbent polymer was measured using a digital force gauge FGP-2 which is a tensile and compression testing machine. As the tip penetrated, the peak value of the force (N) applied to the tip was measured three times according to the following conditions, and the arithmetic mean value thereof was determined as the gel strength (unit: N).

Tip size: terminal diameter 10±0.1 mm
Beaker size: 50±0.1 mm
Penetration speed: 500±0.5 mm/min

With regard to Examples and Comparative Examples, the physical properties of the superabsorbent polymers after surface crosslinking are shown in Table 2 below.

[Table 2]

| Section | CRC (g/g) | AUP (g/g) | Absorption rate (sec) | Gel strength (N) |
|---|---|---|---|---|
| Example 1 | 37.8 | 18.3 | 46 | 0.68 |
| Example 2 | 38.1 | 18.5 | 45 | 0.80 |
| Example 3 | 40.0 | 20.0 | 43 | 0.67 |
| Example 4 | 39.2 | 19.6 | 46 | 0.70 |
| Example 5 | 37.5 | 17.8 | 35 | 0.72 |
| Example 6 | 37.8 | 17.9 | 45 | 0.76 |
| Example 7 | 36.5 | 20.1 | 40 | 0.82 |
| Example 8 | 41.4 | 14.8 | 56 | 0.64 |
| Example 9 | 36.5 | 20.0 | 45 | 0.55 |
| Comparative Example 1 | 37.8 | 18.0 | 48 | 0.43 |
| Comparative Example 2 | 38.5 | 17.5 | 45 | 0.38 |
| Comparative Example 3 | 37.5 | 16.0 | 35 | 0.45 |
| Comparative Example 4 | 36.3 | 19.2 | 62 | 0.35 |
| Comparative Example 5 | 37.2 | 18.9 | 47 | 0.22 |

Referring to the data in Table 2, it was confirmed that the superabsorbent polymers of Examples according to the present invention may have excellent overall absorption performances such as water retention capacity and absorbency under pressure, and may realize a very high absorption rate and excellent gel strength at the same time by using three specific additives in combination during the polymerization.

In contrast, Comparative Examples, in which some of the three additives of the present invention are not included, showed that it is difficult to form the desired crosslinked structure, and accordingly, it is difficult to realize excellent absorption performances and gel strength at the same time, and in particular, gel strength thereof was significantly reduced, as compared with those of Examples.

## Claims

**1.** A method of preparing a superabsorbent polymer, the method comprising the steps of:

forming a water-containing gel polymer including a crosslinked polymer obtained by crosslinking polymerization of a water-soluble ethylenically unsaturated monomer having an acidic group of which at least a part is neutralized, in the presence of an epoxy-based internal crosslinking agent, a chelating agent, and a capsule-type foaming agent;
drying the water-containing gel polymer to form a base polymer powder; and
surface-crosslinking the base polymer powder by heat treatment in the presence of a surface crosslinking agent,
wherein the epoxy-based internal crosslinking agent has a weight average molecular weight of 200 kDa to 1,000 kDa, and
the capsule-type foaming agent has a structure including a core including hydrocarbon and a shell surrounding the core, wherein shell is formed by a thermoplastic resin.

**2.** The method of claim 1, wherein the epoxy-based internal crosslinking agent, the chelating agent, and the capsule-type foaming agent are included in an amount of 0.01 part by weight to 10 parts by weight, 0.01 part by weight to 10 parts by weight, and 0.01 part by weight to 10 parts by weight, respectively, based on 100 parts by weight of the water-soluble ethylenically unsaturated monomer.

**3.** The method of claim 1, wherein the epoxy-based internal crosslinking agent has an epoxy equivalent weight of 100 g/eq to 400 g/eq.

**4.** The method of claim 1, wherein the chelating agent is one or more selected from the group consisting of ethylenediamine tetraacetic acid, diethylenetriamine pentaacetic acid, phenolic acid, citric acid, amino acids and salts thereof.

**5.** The method of claim 1, wherein, in the capsule-type foaming agent, the hydrocarbon is one or more selected from the group consisting of n-propane, n-butane, iso-butane, cyclobutane, n-pentane, iso-pentane, cyclopentane, n-hexane, iso-hexane, cyclohexane, n-heptane, iso-heptane, cycloheptane, n-octane, iso-octane, and cyclooctane, and the thermoplastic resin is a polymer formed from one or more monomers selected from the group consisting of (meth)acrylate, (meth)acrylonitrile, aromatic vinyl, vinyl acetate, vinyl halide, and vinylidene halide.

**6.** The method of claim 1, wherein the crosslinked polymer is crosslinked by further including a branched polymer of poly(meth)acrylic acid including polyalkylene glycol in side chains.

**7.** The method of claim 6, wherein the branched polymer is included in an amount of 0.01 part by weight to 5 parts by weight, based on 100 parts by weight of the water-soluble ethylenically unsaturated monomer.

**8.** The method of claim 1, wherein the drying step is carried out at a temperature of 150°C to 250°C for 10 minutes to 120 minutes.

**9.** The method of claim 1, wherein a gel strength of the superabsorbent polymer is 0.50 N or more, as measured using a tensile and compression testing machine after immersing 2.5 g of the superabsorbent polymer in 50 g of ascorbic acid saline and swelling the superabsorbent polymer in an oven at 40 °C for 24 hours.

**10.** The method of claim 1, wherein an absorption rate of the superabsorbent polymer is 50 seconds or less, as measured according to a vortex method.

**11.** The method of claim 1, wherein a centrifuge retention capacity (CRC) of the superabsorbent polymer is 36.5 g/g or more, as measured according to the EDENA method WSP 241.2.

**12.** The method of claim 1, wherein an absorbency under pressure (AUP) of 0.7 psi of the superabsorbent polymer is 17 g/g or more, as measured according to the EDENAmethod 242.3-10.

## INTERNATIONAL SEARCH REPORT

International application No. **PCT/KR2022/000861**

### A. CLASSIFICATION OF SUBJECT MATTER

**C08J 3/24**(2006.01)i; **C08J 3/12**(2006.01)i; **C08J 3/075**(2006.01)i; **C08J 9/14**(2006.01)i; **C08F 265/06**(2006.01)i; **C08F 220/06**(2006.01)i; **C08F 2/44**(2006.01)i; **C08K 9/10**(2006.01)i

According to International Patent Classification (IPC) or to both national classification and IPC

### B. FIELDS SEARCHED

Minimum documentation searched (classification system followed by classification symbols)

C08J 3/24(2006.01); B01J 20/26(2006.01); B65D 35/38(2006.01); C08F 2/44(2006.01); C08J 3/12(2006.01); C08J 9/14(2006.01); C08K 5/00(2006.01); C11D 17/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(super absorbent resin), 에폭시계 내부 가교제(epoxy internal crosslinking agent), 킬레이트제(chelating agent), 캡슐형 발포제(capsule foaming agent), 에틸렌계 불포화 단량체(ethylenic ally unsaturated monomer)

### C. DOCUMENTS CONSIDERED TO BE RELEVANT

| Category* | Citation of document, with indication, where appropriate, of the relevant passages | Relevant to claim No. |
|---|---|---|
| Y | KR 10-2016-0127742 A (NIPPON SHOKUBAI CO., LTD.) 04 November 2016 (2016-11-04) See paragraphs [0031], [0056], [0059], [0060], [0066], [0075], [0076], [0142], [0152], [0154], [0200], [0261], [0262] and [0306]; and figure 2. | 1-12 |
| Y | KR 10-2020-0075196 A (LG CHEM, LTD.) 26 June 2020 (2020-06-26) See paragraphs [0089], [0094] and [0095]; example 1; and claim 2. | 1-12 |
| A | WO 2009-029087 A2 (ABBOTT LABORATORIES) 05 March 2009 (2009-03-05) See entire document. | 1-12 |
| A | US 2008-0058239 A1 (EVERS, Marc Francois Theophile et al.) 06 March 2008 (2008-03-06) See entire document. | 1-12 |
| A | KR 10-2019-0125027 A (LG CHEM, LTD.) 06 November 2019 (2019-11-06) See entire document. | 1-12 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 May 2022** | **02 May 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2022/000861**

| Patent document cited in search report | Publication date (day/month/year) | Patent family member(s) | Publication date (day/month/year) |
|---|---|---|---|
| KR 10-2016-0127742 A | 04 November 2016 | JP 6441894 B2 | 19 December 2018 |
| | | US 10207250 B2 | 19 February 2019 |
| | | US 2017-0014801 A1 | 19 January 2017 |
| KR 10-2020-0075196 A | 26 June 2020 | None | |
| WO 2009-029087 A2 | 05 March 2009 | JP 2009-542895 A | 03 December 2009 |
| | | US 2008-0089940 A1 | 17 April 2008 |
| | | US 7988992 B2 | 02 August 2011 |
| US 2008-0058239 A1 | 06 March 2008 | US 2008-0057020 A1 | 06 March 2008 |
| | | US 2008-0058240 A1 | 06 March 2008 |
| | | US 2008-0058241 A1 | 06 March 2008 |
| | | US 2008-0099041 A1 | 01 May 2008 |
| KR 10-2019-0125027 A | 06 November 2019 | JP 2020-528945 A | 01 October 2020 |
| | | JP 6937888 B2 | 22 September 2021 |
| | | US 2020-0270441 A1 | 27 August 2020 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210006720
- KR 1020220006530
- WO 1987003208 A


### Non-patent literature cited in the description


- **ODIAN.** Principle of Polymerization. Wiley, 1981, 203
- **REINHOLD SCHWALM.** UV Coatings: Basics, Recent Developments and New Application. Elsevier, 2007, 115